# EUROPÄISCHE PATENTSCHRIFT

(11) **EP 0 495 461 B1**
(45) Veröffentlichungstag und Bekanntmachung des Hinweises auf die Patenterteilung: **01.05.1996**
(21) Anmeldenummer: 92100521.1
(22) Anmeldetag: 14.01.1992
(51) Int. Cl.: A61B 5/22

(54) **Vorrichtung zum Messen der Kräfte der menschlichen Hand oder ihrer einzelnen Finger**
Device for measuring the strength of the human hand or finger
Dispositif de mesure de la force d'une main ou d'un doigt

(30) Priorität: 14.01.1991 DE 9100382 U
(43) Veröffentlichungstag der Anmeldung: 22.07.1992
(73) Patentinhaber: Weber, Hansjörg, Dr. med. habil., D-01662 Meissen (DE)
(72) Erfinder: Weber, Hansjörg, Dr. med. habil., D-01662 Meissen (DE)
(74) Vertreter: Görg, Klaus, Dipl.-Ing.

(56) Entgegenhaltungen:
- DD-A- 204 206
- US-A- 3 680 386
- MEDICAL AND BIOLOGICAL ENGINEERING AND COMPUTING. Bd. 19, Nr. 1, Januar 1981, STEVENAGE GB Seiten 127 - 128; PRONK UND NIESING: 'Measuring hand-grip force, using a new application of strain gauges.'
- PROCEEDINGS OF THE IEEE NATIONAL AEROSPACE AND ELECTRONICS CONFERENCE Bd. 2,
- 1981, US Seiten 574 - 580; PETROFSKY: 'Digital controlled handgrip dynamometer for isometric performance studies.'

## Beschreibung

Die Erfindung betrifft eine Vorrichtung zum Messen der Kräfte der menschlichen Hand oder ihrer einzelnen Finger mittels eines Kraftmeßaufnehmers, der einen ihn durchdringenden Zugbolzen umfaßt und von einem Gehäuse umgeben ist.

Eine solche Vorrichtung, mit der die Beugekräfte der menschlichen Hand oder des zweiten bis fünften Fingers der verschiedenen Beugewinkel gemessen werden können, ist aus der DD-PS 204 206 bekannt. Die Messung dieser Kräfte wird dadurch bewirkt, daß die Griffteile auf senkrecht zueinander stehenden Linien frei wählbar sind und ein vom zweiten bis fünften Finger erfaßtes, vertikal auf die Höhe jedes Fingers einstellbares Griffteil im Wirkungseingriff mit einem Kraftmeßaufnehmer (Meßwandler) steht. Das bewegliche Griffteil ist mit einem Zugbolzen verbunden, der an seinem entgegengesetzten Ende einen Flansch aufweist, der unter der Kraft einer Druckfeder am zentralen Bereich der Druckmeßdose anliegt, die den Zugbolzen koaxial verkanntungsfrei umfaßt. Eine fehlerfreie Messung der Beugekraft des Daumens ist jedoch mit dieser Vorrichtung nicht möglich.

Diese Messung wird durch eine Vorrichtung (DE-PS 36 34 940) möglich, bei der unter Verwendung der vorbeschriebenen Konstruktion der horizontale und vertikale Winkel der Längsachse eines Handgriffes mit Widerlage zur Längsachse des mit dem Meßwandler im Wirkungseingriff stehenden Zugbolzens und seiner Neigung gegenüber der Horizontalebene in einer zweiten, senkrecht zur ersten befindlichen Vertikalebene durch einen Drehständer und zwei Drehgelenken sowie der Längs- und Querabstand des Handgriffes zu dem mit dem Zugbolzen verbundenen Fingerzuggriff mit Hilfe eines Längs- und eines Quersupports frei einstellbar sind.

Die beschriebenen Vorrichtungen ermöglichen zwar exakte Messungen der Beugekräfte der menschlichen Hand oder einzelner Finger jeweils genau in Wirkungsrichtung der Kraft bei biomechanisch gleichen Bedingungen für Hände unterschiedlicher Größe und bei verschiedenen Beugewinkeln der Finger ein und derselben Hand, gestatten jedoch nicht die Messung der für die verschiedenen Gebiete der Medizin bedeutsamen Streckkräfte der menschlichen Hand oder ihrer einzelnen Finger.

Aus der US-A-3 680 386 ist eine Meßvorrichtung bekannt, mit der für Körperglieder die Größe einer Stoßkraft und auch einer Zugkraft gemessen werden kann.

Es ist Aufgabe der Erfindung, eine Vorrichtung zu schaffen, mit deren Hilfe sowohl die willkürlich erzeugten Beugekräfte, als auch die Streckkräfte der menschlichen Hand oder jedes einzelnen Fingers einschließlich des Daumens auf einfache Weise optimal genau gemessen werden können.

Diese Aufgabe wird erfindungsgemäß durch die Merkmale des Patentanspruchs 1 gelöst.

Diese Lösung hat den Vorteil, daß sowohl Zug- als auch Druckkräfte und damit sowohl die Beuge- als auch die Streckkräfte der Hand oder der einzelnen Finger optimal und mit geringem Aufwand gemessen werden können.

Durch den Einsatz der Reversierscheibe und der Widerlager können auf einfache Weise unter Verwendung des Kraftmeßaufnehmers die erwähnten Zug- und Druckkräfte auf einfache Weise mit geringen Umstellungserfordernissen genau gemessen werden.

Es ist zweckmäßig, dabei für die kraftschlüssige Verbindung das Betätigungsteil mittels einer Stiftverbindung mit der Reversierscheibe zu verbinden, wobei die Stiftverbindung durch den Zugbolzen verläuft, ohne eine Kraft auf diesen zu übertragen, wozu der Stift den Zugbolzen in Axialrichtung des Zugbolzens mit einem gewissen Spiel durchragt. Dadurch erfolgt eine Entkoppelung, die über die Reversierscheibe die Messung in entgegengesetzten Wirkungsrichtungen mit einer Kraftmeßdose erlaubt.

Damit eine möglichst genaue Messung vorgenommen werden kann, ist der Kraftmeßaufnehmer in einer Richtung mit möglichst geringen Berührungsflächen gelagert, damit durch Reibung bedingte Meßungenauigkeiten gering gehalten werden können.

Dabei kann diese Einrichtung eine Distanzhülse mit ringförmigen Rippen sein.

Durch die erfindungsgemäße Lösung ist gewährleistet, daß die zu messenden Kräfte, ausgehend von den Fingern der menschlichen Hand oder der Hand selbst exakt und definiert in die Vorrichtung eingegeben werden können. Die Handstütze verhindert eine Beeinflussung der von den Fingern ausgeübten Kräfte seitens des Armes und des übrigen Körpers. Das Ellbogenwiderlager erlaubt einen Ausschluß des Einflusses von Körperbewegungen über den Arm auf Hand und Finger bei der zu messenden Krafteinwirkung.

Im folgenden soll die Erfindung anhand der beigefügten Zeichnungen näher beschrieben werden. Es zeigt:
- Fig. 1: eine schematische Gesamtseitenansicht einer Meßvorrichtung der Erfindung,

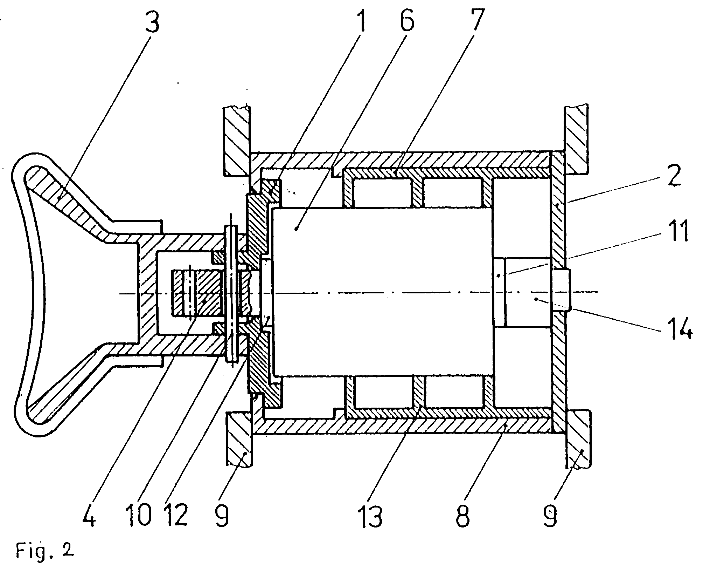

- Fig. 2: einen Längsschnitt eines Teils der Vorrichtung gemäß Fig. 1 mit der Darstellung der Teile für die Messung der Streckkräfte und

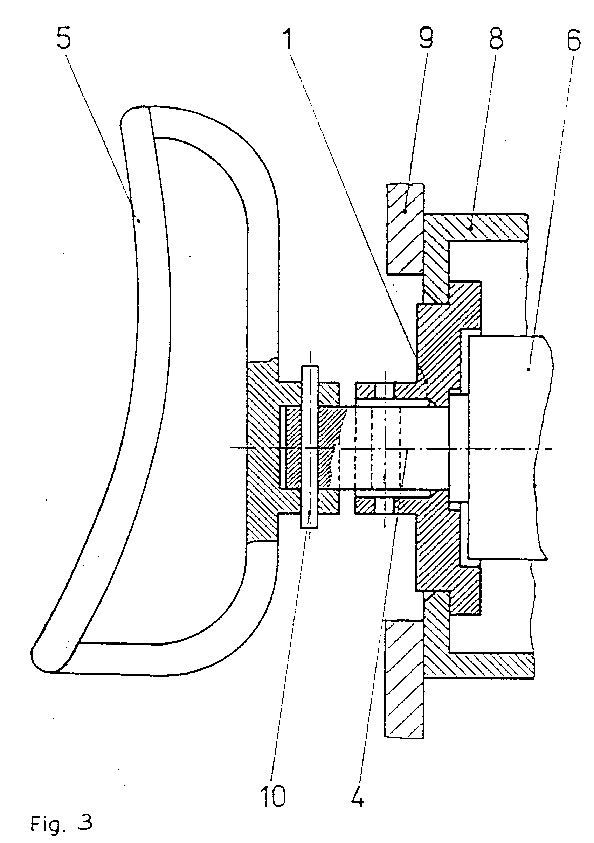

- Fig. 3: einen Längsschnitt eines Teils der Vorrichtung gemäß Fig. 1 mit der Darstellung der Teile für die Messung der Beugekräfte.

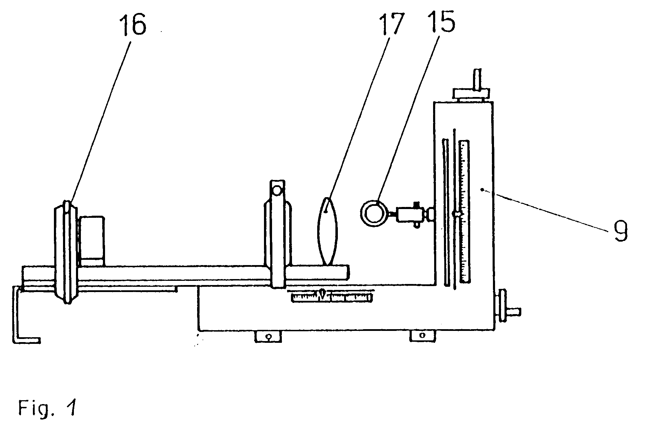

Die Meßvorrichtung gemäß Fig. 1 trägt das Bezugszeichen 9. Diese Vorrichtung ist mit als wesentliche Teile einem Fingergriffteil 15 zum Ansetzen der zu messenden Fingerkräfte, einem Griffteil 17 als Handstütze und einem Widerlager 16 versehen. Die zur Messung der Hand- oder Fingerkräfte gleich große Gegenkraft wird bei Streckkräften am distalen Ende des Oberarms, der sich an dem Widerlager 16 abstützt und bei der Messung der Beugekräfte an dem horizontal beweglichen Griffteil 17 im Bereich der Mittelhand und der Handwurzel erzeugt.

Bei Meßvorrichtungen ohne Griffteil 17 oder Widerlager 16 ist eine spezifische Messung der Beuge- und Streckkräfte der Hand und der einzelnen Finger nicht möglich, da die Gegenkraft durch andere Körperteile wie Oberarm, Schultergürtel, Rumpf und untere Extremitäten erzeugt werden muß.

Nachfolgend wird nun die erfindungsgemäße Vorrichtung an Hand der übrigen Zeichnungen weiter erläutert.

In einem Gehäuse 8 mit Drucklager 2 eines zu Kraftmessungen bestimmten zeichnerisch nur angedeuteten Gerätes 9 sind ein Meßwandler 6 mit Distanzhülse 7, ein Zugbolzen 4 und eine Reversierscheibe 1 so angeordnet, daß sie zur Messung von Zug- und Druckkräften geeignet sind, obwohl der Meßwandler 6 nur auf Druck belastbar ist. Zur Messung der Beugekräfte der Hand oder der einzelnen Finger wird ein Handgriff 5 mit Hilfe eines Stiftes 10 am Zugbolzen 4 befestigt. In Folge der Wirkung der Druckkräfte übt der Flansch 14 des Zugbolzens 4 auf den Flansch 11 des Meßwandlers 6 eine Kraft aus und drückt den Flansch 12 des Meßwandlers 6 gegen die bei Zugkräften im Wirkungszusammenhang mit dem Gehäuse 8 und dem Gerät 9 als Widerlager dienende Reversierscheibe 1, so daß im Meßwandler 6 eine der Zugkraft proportionale Druckspannung entwickelt wird. Um Streckkräfte der Hand oder einzelner Finger zu messen, wird ein Druckstück 3 auf die Reversierscheibe 1 gesetzt und gegen Verschiebung durch einen Stift 10 an der Reversierscheibe gesichert. Dabei sind die entsprechenden Bohrungen in der Reversierscheibe 1 und im Zugbolzen 4 in einem Durchmesser anzubringen, daß die Druckkraft über den Stift 10 auf die Reversierscheibe 1, jedoch nicht auf den Zugbolzen 4 übertragen werden kann.

In Folge der losen Verbindung der Reversierscheibe 1 mit dem Gehäuse 8 kann die Druckkraft vom Druckstück 3 über die Reversierscheibe 1 auf den an der Reversierscheibe 1 anliegenden Flansch 12 des Meßwandlers 6 übertragen werden, wobei der Flansch 14 des Zugbolzens 4 in Verbindung mit dem Drucklager 2 und dem Gerät 9 für Flansch 11 des Meßwandlers 6 als Widerlager dient. Dadurch werden auch bei der Einwirkung von Druckkräften diesen proportionale Druckspannungen im Meßwandler 6 erzeugt.

Die dem Meßwandler 6 gegenüberliegende Seite der Reversierscheibe 1 ist so gestaltet, daß die Reversierscheibe 1 an der Stirnseite des Flansches 12 des Meßwandlers 6 anliegt und dadurch eine funktionsgerechte exakte Krafteinleitung in den Meßwandler 6 gewährleistet wird. Möglichst geringe Berührungsflächen zwischen Meßwandler 6, den ringförmigen Rippen 13 der Distanzhülse 7, Meßwandler 6 und Zugbolzen 4, Drucklager 2 und Zugbolzen 4, Reversierscheibe 1 und Zugbolzen 4 sowie Reversierscheibe 1 und Gehäuse 8 bewirken eine weitgehende Verringerung der Reibungsverluste und damit eine nur unwesentliche Beeinträchtigung der Güte der Messung. Drucklager 2, Reversierscheibe 1 und Distanzhülse 7 sichern sowohl bei Zug- als auch Druckkraftmessung und damit sowohl bei der Messung der Beuge- als auch der Streckkräfte der Hand oder jedes einzelnen Fingers eine exakte Lagerung von Zugbolzen 4 und Meßwandler 6.

Das Druckstück 3 oder der Handgriff 5 können durch Kraftaufnahmeelemente ersetzt werden, die der jeweiligen Meßaufgabe an der Hand oder am einzelnen Finger entsprechen, wie Hand-, Finger- oder Daumengriffteile bei der Messung der Beugekräfte oder entsprechende Druckstücke bei der Messung der Streckkräfte.

Die Umstellung der Vorrichtung von einen auf den anderen Anwendungsfall erfolgt einfach und schnell durch den Austausch der Kraftaufnahmeelemente.

## Patentansprüche

1. Vorrichtung zum Messen der Kräfte der menschlichen Hand oder ihrer einzelnen Finger mittels eines Kraftmeßaufnehmers (6), der einen ihn durchdringenden Zugbolzen (4) umfaßt und von einem Gehäuse (8) umgeben ist, dadurch **gekennzeichnet**,
daß ein Betätigungsteil (5) zur Aufbringung von Beugekräften oder Zugkräften kraftschlüssig mit dem Zugbolzen (4) verbunden ist,
daß eine Handstütze (17) als ein Widerlager zur Aufbringung von Beugekräften oder Zugkräften vorgesehen ist,
daß ein Betätigungsteil (3) zur Aufbringung von Streckkräften oder Druckkräften kraftschlüssig mit einer Reversierscheibe (1) verbunden ist,
daß ein Ellbogenwiderlager (16) zur Aufbringung von Streckkräften oder Druckkräften vorgesehen ist, daß sich die Reversierscheibe bei aufgebrachten Beugekräften oder Zugkräften am Gehäuse (8) abstützt, wobei der Zugbolzen (4) in axialer Richtung relativ zu der Reversierscheibe (1) und dem Kraftmeßaufnehmer (6) verschiebbar ist,
und bei aufgebrachten Streckkräften oder Druckkräften am Kraftmeßaufnehmer (6) abstützt, wobei die Reversierscheibe (1) und der Kraftmeßaufnehmer (6) relativ zu dem Zugbolzen (4) und dem Gehäuse (8) verschiebbar sind.

2. Vorrichtung nach Anspruch 1, dadurch **gekennzeichnet**, daß für die kraftschlüssige Verbindung das Betätigungsteil (3) mittels einer Stiftverbindung mit der Reversierscheibe (1) verbunden ist, wobei die Stiftverbindung durch den Zugbolzen (4) verläuft, ohne eine Kraft auf diesen zu übertragen.

3. Vorrichtung nach Anspruch 1, dadurch **gekennzeichnet**, daß der Kraftmeßaufnehmer in einer Einrichtung mit möglichst geringen Berührungsflächen gelagert ist.

4. Vorrichtung nach Anspruch 3, dadurch **gekennzeichnet**, daß die Einrichtung eine Distanzhülse mit ringförmigen Rippen ist.

5. Vorrichtung nach Anspruch 1, dadurch **gekennzeichnet**, daß als Widerlager zum Aufnehmen der Reaktionskräfte beim Messen der Kräfte der menschlichen Hand oder ihrer einzelnen Finger mindestens ein Abstützelement vorgesehen ist.

## Claims

1. Device for measuring the forces of the human hand or of its individual fingers by means of a dynamometric pick-up (6) which comprises a tension bolt (4) penetrating it and is surrounded by a housing (8), characterised in
that an actuating part (5) for the application of bending forces or tensile forces is connected in force-locking manner to the tension bolt (4), that a hand support (17) is provided as an abutment for the application of bending forces or tensile forces,
that an actuating part (3) for the application of stretching forces or compression forces is connected in force-locking manner to a reversing disk (1), that an elbow abutment (16) for the application of stretching forces or compression forces is provided,
that the reversing disk is supported on application of bending forces or tensile forces against the housing (8), the tension bolt (4) being displaceable in the axial direction relative to the reversing disk (1) and the dynamometric pick-up (6),
and on application of stretching forces or compression forces is supported on the dynamometric pick-up (6), the reversing disk (1) and the dynamometric pick-up(6) being displaceable relative to the tension bolt (4) and the housing (8).

2. Device according to claim 1, characterised in that for the force-locking connection, the actuating part (3) is connected by means of a pin connection to the reversing disk (1), the pin connection extending through the tension bolt (4) without transmitting a force to the latter.

3. Device according to claim 1, characterised in that the dynamometric pick-up is mounted in an arrangement with the smallest possible contact surfaces.

4. Device according to claim 3, characterised in that the arrangement is a spacer sleeve with annular ribs.

5. Device according to claim 1, characterised in that as abutment for receiving the reaction forces when measuring the forces of the human hand or of its individual fingers at least one support element is provided.

## Revendications

1. Dispositif destiné à mesurer les forces de la main humaine ou de ses doigts individuels, au moyen d'un capteur de mesure de force (6), qui comprend une broche de traction (4) le traversant, et qui est entouré par un carter (8), caractérisé
en ce qu'une pièce d'actionnement (5) destinée à appliquer des forces de flexion ou des forces de traction, est reliée par une liaison de transmission d'efforts, à la broche de traction (4),
en ce qu'il est prévu un support de main (17) en tant que butée d'appui pour l'application de forces de flexion ou de forces de traction,
en ce qu'une pièce d'actionnement (3) destinée à appliquer des forces d'extension ou des forces de compression est reliée par une liaison de transmission d'efforts, à un disque d'inversion (1),
en ce qu' il est prévu une butée d'appui de coude (16) destinée à l'application de forces extension ou de forces de compression,
et en ce que le disque d'inversion s'appuie sur le carter (8) lorsque les forces de flexion ou les forces de traction sont appliquées, la broche de de traction (4) étant susceptible de coulisser dans la direction axiale par rapport au disque d'inversion (1) et au capteur de mesure de force (6),
et s'appuie sur le capteur de mesure de force (6) lorsque des forces d'extension ou les forces de compression sont appliquées, le disque d'inversion (1) et le capteur de mesure de force (6) étant susceptibles de coulisseur par rapport à la broche de traction (4) et au carter (8).

2. Dispositif selon la revendication 1, caractérisé en ce que pour la liaison de transmission d'efforts, l'élément d'actionnement (3) est relié au disque d'inversion (1) au moyen d'une liaison par goupille, la liaison par goupille d'étendant au travers de la broche de traction (4) sans transmettre de force à celle-ci.

3. Dispositif selon la revendication 1, caractérisé en ce que le capteur de mesure de force est monté dans un système présentant des surfaces de contact les plus réduites possible.

4. Dispositif selon la revendication 3, caractérisé en ce que le système est constitué par une douille entretoise à nervures annulaires.

5. Dispositif selon la revendication 1, caractérisé en ce qu'en guise de butée d'appui destinée à absorber les forces de réaction lors de la mesure des forces de la main humaine ou de ses doigts individuels, est prévu au moins un élément de support d'appui.
